# EUROPEAN PATENT APPLICATION

(11) **EP 3 932 393 A1**
(43) Date of publication of application: **05.01.2022**
(21) Application number: 21182357.0
(22) Date of filing: 29.06.2021
(51) Int. Cl.: A61K 9/00

(54) **PHARMACEUTICAL FORMULATION**

(30) Priority: 03.07.2020 GB 202010230
(71) Applicant: Alkaloid AD Skopje, 1000 Skopje (MK)
(72) Inventor: Memed Sejfulah, Suzan, 1000 Skopje (MK); Trajanovska, Eleonora, 1000 Skopje (MK); Atanasova, Ana, 1000 Skopje (MK); Jovanovikj, Frosina, 1000 Skopje (MK)
(74) Representative: CSY London

(57) **Abstract**

The present invention relates to a formulation of ketamine, to a process for the manufacture of such a formulation, and to medical uses of such a formulation. The formulations show enhanced palatability and storage stability.

## Description

### Technical Field

The present invention relates to a formulation of ketamine, to a process for the manufacture of such a formulation, and to medical uses of such a formulation.

### Background of the Invention

Ketamine is a versatile medicine and possibly the most widely used anaesthetic in the world. Ketamine is also effective for relieving pain. It is listed as an essential medicine, meaning that it should be available at all times in adequate quantities to meet health care needs. It was added to the WHO Model List of Essential Medicines in 1985 and is also on the Model List of Essential Medicines for Children.4 Ketamine is extremely safe because it does not depress breathing or blood pressure and does not require expensive patient-monitoring. This means that oral ketamine solution is easy and safe to self-administer. The risk of fatal intoxication with oral ketamine solution is extremely low.

Ketamine has been used world-wide for over 50 years. It is an arylcycloalkylamine related to phencyclidine and was introduced into clinical practice in the 1960s. It was first licensed as an injectable dissociative anaesthetic in the EU in 1968 and in the USA in 1970. Ketamine is a phenylpiperidine derivative structurally related to phencyclidine with 2(2-chlorophenyl)-2-(methylamino)-cyclohexanone as its chemical structure. The chiral centre on the C-2 atom of the ketamine cyclohexane ring gives rise to two stereoisomers, S(+) - and R(-)- ketamine. The more active enantiomer, S(+)-ketamine, is two times stronger than the racemic form, and four times than the R(-)-ketamine isomer.

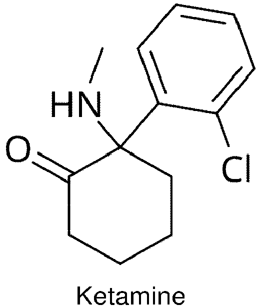

Ketamine is mainly metabolised by the liver to the active metabolite norketamine. Rapid and extensive first pass metabolism results in 80% of oral ketamine being converted to norketamine before it reaches the systemic circulation. Norketamine is also a non-competitive NMDA antagonist, but much less potent than ketamine. In essence, unlike ketamine injection, ketamine when given orally is the prodrug for norketamine.

The S-ketamine enantiomer (or S-(+)-ketamine or esketamine) has higher potency or affinity for the NMDA receptor and thus potentially allowing for lower effective dosages; and is available for medical use, administered either IV (intravenously) or IM (intramuscularly), under the brand name KETANEST S.

Ketamine is typically administered by intravenous injection. Oral dosage forms of ketamine are known, for example from WO2015158854. This discloses an oral dosage form comprising ketamine, which provides a specific *in vitro* release of ketamine. Ketamine oral solution (usually 50mg/5mls) is available as a "special" (i.e. and individually prepared formulations of existing drugs made for a specific patient) product in the National Health Service of the United Kingdom and can be used for both oral and buccal administration.

A problem that remains is that ketamine has an extremely bitter taste, which is not well masked by conventional flavouring agents. In the past, ketamine has been formulated as a suspension for oral administration, but this suffers from the drawback of inhomogeneity, with lumps of flavour-masking agent tending to aggregate.

The prior art has shown extensive use of one or a combination of different flavouring methodologies to mask the bitter taste of drugs. For example, a flavour can be selected that complements the taste of the preparation, or a flavour with a longer intensity and stronger taste than the drug can be used. High levels of sweetening agents are often used to overwhelm bitterness with sweetness. The taste buds may also be anesthetized by menthol or mint flavours. These approaches are generally not very effective in masking the taste of a bitter drug, and a flavouring system that works with one drug often does not apply to another drug.

This invention addresses these and other problems of the prior art.

### Summary of the Invention

According to a first embodiment, the invention relates to a liquid pharmaceutical formulation comprising
i. an active ingredient selected from ketamine and esketamine or a pharmaceutically acceptable salt thereof;
ii. a cellulose ether;
iii. glycerol; and
iv water.

According to a further embodiment, the invention provides a liquid pharmaceutical formulation comprising an active ingredient selected from ketamine and esketamine or a pharmaceutically acceptable salt thereof; cellulose ether in an amount of from 1.0 - 15.0 %(w/w)
a preservative in an amount of from 0.01 - 0.6%
one or more polyols in an amount of from 5.0 - 30.0%
and balance water, wherein the formulation has an acceptable taste

According to a further embodiment, there is provided a pharmaceutical composition of the invention for oral administration to a human or animal.

According to a further embodiment there is provided a pharmaceutical composition of the invention for use in the treatment of pain.

According to a further embodiment there is provided a pharmaceutical composition of the invention for use in the treatment of depression.

According to a further embodiment there is provided a method of treatment of pain comprising administering to a patient in need thereof an effective amount of the pharmaceutical composition of the invention.

According to a further embodiment there is provided a method of treatment of depression comprising administering to a patient in need thereof an effective amount of the pharmaceutical composition of the invention.

### Detailed Description of the Invention

The active ingredient used in the formulations of the present invention is selected from ketamine, including isomeric mixtures thereof in all ratios, and single enantiomers, especially esketamine.

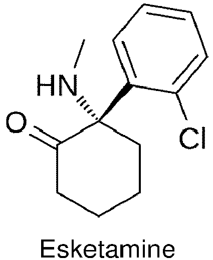

The active ingredient may be present as free base, or alternatively may be present as a pharmaceutically acceptable salt. As used herein, the term "pharmaceutically acceptable salt" includes salts of organic or inorganic acids, such as hydrochloride, hydrobromide, tartrate, mesylate, acetate, maleate, and oxalate. Preferably, the hydrochloride is used.

The formulations of the present invention are liquids. The term "liquid" encompasses suspensions, solutions and the like. Preferably, the formulations of the invention are solutions, and not suspensions. This in part distinguishes the formulations of the present invention from those known in the art.

The formulations of the present invention are intended to be administered orally (i.e. by ingestion) or via enteral feeding tubes. As used herein, the term "administering" means providing a formulation to a subject, and includes, but is not limited to, administering by a medical professional and self-administering. Administration of a formulation of the invention, a compound or an agent to a subject can be carried out using one of a variety of methods known to those skilled in the art. Administering can also be performed, for example, once, a plurality of times, and/or over one or more extended periods.

The formulations of the present invention comprise a cellulose ether. A "cellulose ether," as used herein, is an ether-linked derivative, either partial or complete, of cellulose. Cellulose ether is produced from cellulose pulp, typically obtained from wood or cotton. The cellulose pulp is converted into alkaline cellulose by alkalizing the cellulose pulp with an alkali hydroxide, and then etherifying the alkalized cellulose in a dry, gas-phase or slurry process with one or more etherifying agents The molecular weight of these cellulose ethers can then be reduced by depolymerizing the cellulose ether with an acid, such as hydrogen chloride, and optionally neutralizing the depolymerized cellulose ether with a basic compound, such as anhydrous sodium bicarbonate. Alternatively, the cellulose ether may be depolymerized by way of acid catalysed degradation, oxidative degradation, degradation by high-energy radiation, and degradation by way of microorganisms or enzymes.

The cellulose ether is preferably a water-soluble cellulose ether. A water-soluble cellulose ether is a cellulose ether that prior to the partial depolymerization has a solubility in water of at least 2 grams in 100 grams of distilled water at 25°C and 1 atmosphere. Nonlimiting examples of water soluble cellulose ethers include carboxy- C₁-C₃-alkyl celluloses, such as carboxymethyl celluloses; carboxy- C₁-C₃-alkyl hydroxy- C₁-C₃-alkyl celluloses, such as carboxymethyl hydroxyethyl celluloses; C₁-C₃-alkyl celluloses, such as methylcelluloses; C₁-C₃-alkyl hydroxy-C₁₋₃-alkyl celluloses, such as hydroxyethyl methylcelluloses, hydroxypropyl methylcelluloses or ethyl hydroxyethyl celluloses; hydroxy-C₁₋₃-alkyl celluloses, such as hydroxyethyl celluloses or hydroxypropyl celluloses; mixed hydroxy- C₁-C₃-alkyl celluloses, such as hydroxyethyl hydroxypropyl celluloses, mixed C₁-C₃-alkyl celluloses, such as methyl ethyl celluloses, or alkoxy hydroxyethyl hydroxypropyl celluloses, the alkoxy group being straight-chain or branched and containing 2 to 8 carbon atoms.

In a preferred embodiment, the cellulose ether is selected from methylcellulose, hydroxyethyl cellulose, hydroxypropyl cellulose, hydroxyethyl methyl cellulose, hydroxypropyl methyl cellulose, hydroxy butyl methyl cellulose, ethyl hydroxyethyl cellulose, carboxymethyl cellulose, and carboxymethyl hydroxyethyl cellulose. In a very preferred embodiment, the cellulose ether is hydroxyethyl cellulose.

Preferably, the cellulose ether is present in an amount of from 0.1 mg/mL to 2.5 mg/mL. Amounts in excess of 2.5 mg/mL have been found to resultant in compositions which are unacceptable in terms of palatability. More preferably, the cellulose ether is present in an amount of from 0.2 mg/mL to 2 mg/mL. Still more preferably, the cellulose ether is present in an amount of from 0.5 mg/mL to 1.5 mg/mL. Most preferably, the cellulose ether is present in an amount of about 1 mg/mL.

As used herein, the term "polyol" includes any compound having at least one hydroxyl group on each of two adjacent carbon atoms that are not in trans configuration relative to each other. The polyols can be linear or cyclic, substituted or unsubstituted, or mixtures thereof, so long as the resultant complex is water soluble and pharmaceutically acceptable. Examples of such compounds include sugars, sugar alcohols, sugar acids and uronic acids. Preferred polyols are sugars, sugar alcohols and sugar acids, including, but not limited to mannitol, glycerol, xylitol, sorbitol and propylene glycol. An especially preferred polyol is glycerol.

Preferably, the polyol is present in the formulation in an amount of from 0.1 to 20 mg/mL, more preferably from 1 to 10 mg/mL, still more preferably from 3 to 7 mg/mL, most preferably about 5 mg/mL.

In some embodiments, the formulations of the present invention comprise a pH adjusting agent or buffering agent. A buffering agent is used in the formulation of present invention to stabilize the formulation against chemical degradation that might occur due to pH drift. The buffering agents used in the formulation of present invention can be citrates, acetates, phosphates other organic buffers and the likes. The preferred pH adjusting agent used in the present invention is citric acid monohydrate.

Preferably, the pH of the liquid is between 2 and 5, preferably between 3 and 4.5, more preferably between 4.0 and 4.4, most preferably between 4.10 and 4.20.

Preferably, the viscosity of formulation is below 2 cPs, more preferably between 1.4 and 2.0 cPs, still more preferably between 1.6 and 1.7 cPs.

In some embodiments, the formulations of the present invention comprise a preservative. Suitable preservatives are preferably those selected from the group consisting of benzalkonium chloride, benzethonium chloride, benzoic acid, sodium benzoate, benzyl alcohol, bronopol, cetrimide, cetylpyridinium chloride, chlorhexidine, chlorbutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, sodium propionate, thimerosal, methyl paraben, ethyl paraben, propyl paraben, butyl paraben, isobutyl paraben, benzyl paraben, sorbic acid, and potassium sorbate. A particularly preferred preservative is sodium benzoate. Preferably, the preservative is present in an amount sufficient to inhibit microbiological deterioration of the formulation. Preferably, the preservative is present in an amount of between 0.05 mg/mL and 10 mg/mL, preferably between 0.1 mg/mL and 8 mg/mL more preferably between 0.2 - 5.0 mg/ml, such as about 4 mg/mL.

In some embodiments, the formulations of the present invention comprise one or more sweeteners to further improve their palatability. Preferably, the sweetener is selected from the group including sucralose, acesulfame, aspartame, saccharine, alitame, cyclamate, neohesperidine dihydrochalcone, thaumatina and mixtures thereof. Most preferably, the sweetener is sucralose. When present, such a sweetener is used in an amount of from 0.1 mg/mL to 20 mg/mL, preferably from 0.5 mg/mL to 10 mg/mL, such as about 1 mg/mL.

In some embodiments, the formulations of the present invention comprise one or more flavouring agents. Such flavouring agents are known to those skilled in the pharmaceutical art, and include mint, lemon, raspberry, peach, strawberry and the like. Both natural, nature-identical and synthetic flavouring agents may be used. In contrast to the prior art, special taste-masking additives (such as anethole) are unnecessary in the present formulations.

According to another aspect, the invention relates to a process for the preparation of a formulation as described herein comprising the steps of:
i. forming an aqueous mixture of a cellulose ether and glycerol and optionally a preservative;
ii. adding ketamine or a salt thereof to the aqueous mixture of step i;
iii. optionally adding one or more of a pH adjusting agent, sweetener and flavouring agent.

The process may include further steps customary in the pharmaceutical industry, such as filtration, sterilisation and packaging (e.g. in a bottle).

The invention also encompasses methods of treatment using the formulations hereinbefore described.

According to one aspect, the formulations of the invention are used for the induction of anaesthesia.

According to a further aspect, the formulations of the invention are used in pain management alone and as an adjuvant in treatment resistant pain no longer responding adequately to strong opioids. The invention can alleviate pain from many causes, including but not limited to shock; limb amputation; severe chemical or thermal burn injury; sprains, ligament tears, fractures, wounds and other tissue injuries; dental surgery, procedures and maladies; labour and delivery; migraine; during physical therapy; post-operative pain; radiation poisoning; cancer; acquired immunodeficiency syndrome (AIDS); epidural (or peridural) fibrosis; failed back surgery and failed laminectomy; sciatica; painful sickle cell crisis; arthritis; autoimmune disease; intractable bladder pain; neuropathic pain; and the like.

According to a further aspect, the formulations of the invention are used in the treatment of depression. As used herein, the term "depression" shall be defined to include major depressive disorder, unipolar depression, treatment-refractory depression, resistant depression, anxious depression, bipolar depression and dysthymia (also referred to as dysthymic disorder). Preferably, the depression is major depressive disorder, unipolar depression, treatment-refractory depression, resistant depression, anxious depression or bipolar depression.

As used herein, the term "treatment-refractory or treatment-resistant depression" and the abbreviation "TRD" shall be defined as major depressive disorder that does not respond to adequate courses of at least two antidepressants, preferably two or more antidepressants, more preferably two to three, antidepressants.

Any chronic, treatment-resistant depression may be treated by the methods described herein. Such depression may include but is not limited to any of: major depressive disorder, single episode, recurrent major depressive disorder-unipolar depression, seasonal affective disorder-winter depression, bipolar mood disorder-bipolar depression, mood disorder due to a general medical condition-with major depressive-like episode, or mood disorder due to a general medical condition-with depressive features, wherein those disorders are resistant to treatment in a given patient. Thus, any patient that presents one of those disorders and who has not responded to an adequate trial of one antidepressant in the current episode and has recurrent or chronic depressive symptoms for greater than 2 years can be treated by the methods of the invention.

The dosage of the formulation of the invention will depend on the individual patient and is titrated according to clinical response. Typical individual dosages range from 5 mg to 100 mg of active ingredient. Administration may be daily, twice daily or multiple times daily.

### Examples

Preparation of Formulation According to the Invention and EU standards of GMDP

### Step 1 - weighing

All ingredients are weighed separately and checked qualitatively against the manufacturing formula.

### Step 2 - mixing

Water (70 kg) is added in a double jacket vessel with stirrer OLSA PCBS 200 litres; pH 5.0 - 7.0; temperature 18 - 30 °C.

### Step 3 - mixing

Previously weighed and checked hydroxyethyl cellulose (0.100 kg) and glycerol (0.500 kg) are dispersed in 1 litre stainless steel vessel, transferred in the double jacket vessel of step 2 and mixed for five minutes at 1000 RPM. Sodium benzoate (0.400 kg) was added, and mixed for 5 minutes at 1000 RPM.

### Step 4 - dissolving active ingredient

1.153 kg of ketamine hydrochloride was added to the mixture of step 3, and mixed for 10 minutes at 1000 RPM.

### Step 5 - mixing and pH adjustment

Citric acid monohydrate (0.180 kg), sucralose granular (0.100 kg), and strawberry flavour 054267 AP0551 Firmenich SA, Geneva, Switzerland (0.150 kg) were added to the mixture of step 4, and mixed for 10 minutes at 1000 RPM.

### Step 6 - adjusting to final volume

Volume of solution from step 5 was adjusted to 100 L.

### Step 7 - filtration

The solution of step 6 is filtered through a stainless steel 18 µm filter.

### Step 8 - filling and sealing

The solution was filled into 125 mL type III amber glass bottles, and marked with batch number and expiry date. Screw cap PP 28 child-resistant tamper-evident were fitted.

### Step 9 - packaging

The bottles of step 8 were packaged in printed cardboard boxes.

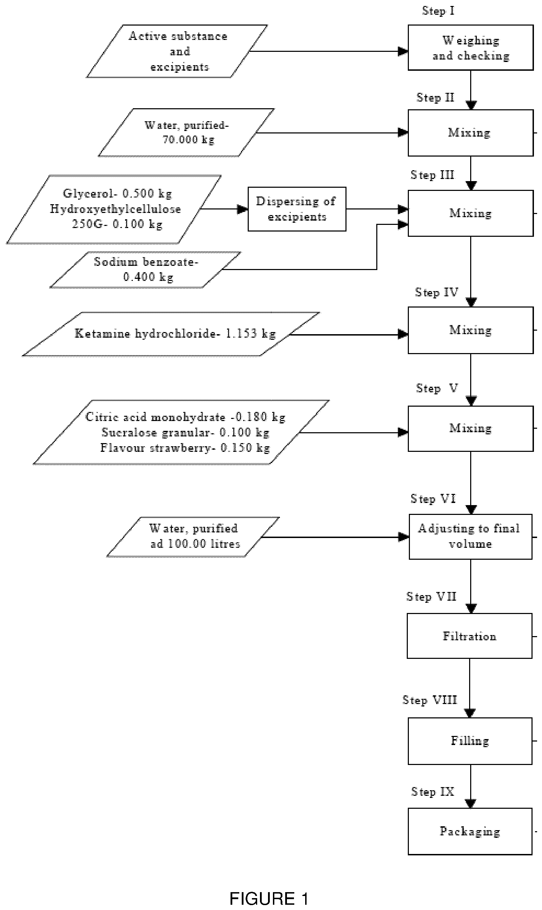

Figure 1 illustrates the manufacturing process.

### Preliminary Organoleptic Assessment

In order to choose the viscosity building agent three commonly used pharmaceutical excipients were used: maltitol liquid, sorbitol liquid non-crystallising and hydroxyethylcellulose 250 G. The rest of the excipients in the formulation were kept constant. The formulations were tested for the physicochemical parameters pH, viscosity and relative density.

The compositions of three test formulations are shown in Table 1.

**Table 1**

| **Ingredient** | **Example** | | |
|---|---|---|---|
| | Comparative Example 1 | Comparative Example 2 | Example 1 |
| Ketamine, HCl (free base equivalent) | 11.534mg | 11.534mg | 11.534mg |
| Maltitol, liquid | 50.0 mg | - | - |
| Sorbitol | - | 30.00 mg | - |
| Hydroxyethylcellulose 250 G | - | - | 1.00 mg |
| Glycerol | - | 10.00 mg | 10.00 mg |
| Sucralose granular | 1.00 mg | 1.00 mg | 1.00 mg |
| Sodium benzoate | 4.00 mg | 4.00 mg | 4.00 mg |
| Citric acid monohydrate | 1.80 mg | 1.80 mg | 1.80 mg |
| Strawberry flavour¹ | 1.50 mg | 1.50 mg | 1.50 mg |
| Water | To 1 mL | To 1 mL | To 1 MI |

| **Parameters** | | | |
|---|---|---|---|
| pH value | 4.28 | 4.27 | 4.18 |
| Viscosity (cP) | 1.60 | 1.34 | 1.67 |
| Relative density | 1.0171 | 1.0132 | 1.0076 |

| **Palatability results** | | | |
|---|---|---|---|
| Organoleptic evaluation | Acceptable | Unacceptable | Acceptable |

| | | | |
|---|---|---|---|
| 1: Strawberry Flavour 054267 AP0551 Firmenich SA, Geneva, Switzerland | | | |

### Palatability Evaluation

Since the product is intended for enteral administration to patients' optimization of organoleptic properties like acceptability of Ketamine 10mg/ml Sugar Free oral solution, 100ml was evaluated. When dissolved in water Ketamine hydrochloride forms clear odourless solution with a pronounced bitter taste. The purpose of the pharmaceutical development was to mask the bitter taste of the drug in order to make it more acceptable to the patients.

Flavours and sweeteners are commonly used in pharmaceutical oral solutions in order to mask drug bitterness and to make the formulation more palatable. For that purpose, three different formulations were tested. Regarding the choice of the flavour a test trial was design and organoleptic evaluation was performed by ten persons. The initial palatability study was conducted by selection between formulations prepared with eight different flavours: raspberry, golden syrup, peach, vanilla, apple, tropical, strawberry and lemon in the recommended concentration of 1.5mg/1ml.

Considering the obtained results from the test trials with organoleptic evaluation, three flavours were chosen as most acceptable: raspberry, peach and strawberry. The compositions are shown in table 2, and the results of the evaluation are shown in Table 3.

**Table 2**

| **Ingredient** | **Example** | | |
|---|---|---|---|
| | Comparative Example 3 | Example 2 | Example 3 |
| Ketamine, HCl | 11,534mg | 11,534mg | 11,534mg |
| Maltitol, liquid | 50.0 mg | - | - |
| Hydroxyethylcellulose 250 G | - | 1.00 mg | 1.00 mg |
| Glycerol | - | 5.00 mg | 5.00 mg |
| Sucralose granular | 1.00 mg | 1.00 mg | 1.00 mg |
| Sodium benzoate | 3.00 mg | 3.00 mg | 3.00 mg |
| Citric acid monohydrate | 2.5 mg | 2.5 mg | 2.5 mg |
| Masking flavour 501438 AP1051¹ | 0.1 mg | 0.1 mg | 0.1 mg |
| Flavours Raspberry flavour Peach flavour Strawberry flavour | 1.50 mg | 1.50 mg | 1.50 mg |
| Water | To 1 mL | To 1 mL | To 1 MI |

| | | | |
|---|---|---|---|
| 1 - preparation of *cis*-Anethole (Firmenich SA, Geneva, Switzerland) | | | |

### Organoleptic evaluation of three different formulations and flavours

**Table 3**

| | Comparative Example 3 | | | Example 2 | | | Example 3 | | |
|---|---|---|---|---|---|---|---|---|---|
| Volunteer | Raspberry | Peach | Strawberry | Raspberry | Peach | Strawberry | Raspberry | Peach | Strawberry |
| 1 | 0 | 2 | 2 | 1 | 1 | 2 | 2 | 1 | 2 |
| 2 | 1 | 0 | 0 | 2 | 0 | 2 | 0 | 2 | 2 |
| 3 | 0 | 1 | 2 | 0 | 2 | 2 | 2 | 0 | 1 |
| 4 | 2 | 0 | 0 | 2 | 1 | 2 | 0 | 2 | 2 |
| 5 | 0 | 1 | 2 | 1 | 0 | 2 | 1 | 2 | 2 |
| 6 | 1 | 0 | 0 | 0 | 2 | 2 | 2 | 0 | 2 |
| 7 | 0 | 2 | 0 | 0 | 1 | 2 | 0 | 2 | 2 |
| 8 | 0 | 0 | 1 | 2 | 2 | 2 | 2 | 2 | 2 |
| 9 | 2 | 0 | 1 | 0 | 2 | 1 | 0 | 0 | 2 |
| 10 | 0 | 0 | 2 | 1 | 0 | 2 | 2 | 1 | 2 |
| Total | 6 | 6 | 10 | 9 | 10 | 19 | 11 | 12 | 19 |
| Total for Example | **22** | | | **38** | | | **42** | | |

| | | | | | | | | | |
|---|---|---|---|---|---|---|---|---|---|
| SCORING SYSTEM: Unacceptable - 0; Not Pleasant - 1; Acceptable - 2 | | | | | | | | | |

The results in Table 3 demonstrates that examples of the invention show superior palatability compared to conventional formulations of ketamine, in conjunction with a range of flavours. Moreover, there is no need to add a taste masking additive, as Example 3 (lacking taste masking additive) shows slightly better palatability compared to Example 2 (having taste masking additive).

## Claims

1. A liquid pharmaceutical formulation comprising
i. an active ingredient selected from ketamine and esketamine or a pharmaceutically acceptable salt thereof;
ii. a cellulose ether;
iii. glycerol; and
iv water.

2. A pharmaceutical formulation according to claim 1 wherein the cellulose ether is a non-ionic cellulose ether selected from the group consisting of methyl cellulose, hydroxyethyl cellulose, methyl hydroxyethyl cellulose, ethyl hydroxyethyl cellulose, methylethyl hydroxyethyl cellulose, hydroxypropyl hydroxyethyl cellulose, methyl hydroxypropyl hydroxyethyl cellulose, hydroxypropyl cellulose, methyl hydroxypropyl cellulose, and ethyl hydroxypropyl cellulose or mixtures thereof, preferably wherein the cellulose ether is hydroxyethyl cellulose.

3. A pharmaceutical formulation according to any one of claims 1 or 2 wherein the cellulose ether is present in an amount of from 0.1 mg/mL to 2 mg/mL, preferably wherein the cellulose ether is present in an amount of from 0.5 mg/mL to 1.5 mg/mL.

4. A pharmaceutical formulation according to any preceding claim wherein the ketamine or esketamine are present in an amount of from 1 mg/mL to 50 mg/mL, preferably wherein the ketamine or esketamine are present in an amount of from 5 mg/mL to 15 mg/mL.

5. A pharmaceutical formulation according to any preceding claim wherein the glycerol is present in an amount of from 5 mg/mL to 15 mg/mL.

6. A pharmaceutical formulation according to any preceding claim further comprising a pH adjusting agent, preferably wherein the pH adjustment agent is citric acid.

7. A pharmaceutical formulation according to any preceding claim wherein the pH of the liquid is between 2 and 5, preferably between 3 and 4.5

8. A pharmaceutical formulation according to claim 6 or 7, wherein the pH adjustment agent is present in an amount of from 5 mg/mL to 15 mg/mL.

9. A pharmaceutical formulation according to any preceding claim further comprising a preservative, preferably wherein the preservative is sodium benzoate.

10. A pharmaceutical formulation according to any preceding claim further comprising a sweetener, preferably wherein the sweetener is sucralose.

11. A pharmaceutical formulation according to any preceding claim further comprising a flavouring agent, preferably wherein the flavouring agent is selected from raspberry, peach, strawberry, mint, lemon, cherry, grape, orange, melon, banana and mint.

12. A pharmaceutical formulation according to any preceding claim wherein the viscosity of formulation is below 2 cPs, preferably between 1.4 and 2.0 cPs.

13. A pharmaceutical formulation as claimed in any one of claims 1 to 12 for oral administration to a human or animal.

14. A pharmaceutical composition as claimed in any one of claims 1 to 12 for induction and/or maintenance of anaesthesia.

15. A pharmaceutical composition as claimed in any one of claims 1 to 12 for use in the treatment of pain, preferably wherein the pain is resistant pain no longer responding adequately to strong opioids, or for the treatment of depression.
